# EUROPEAN PATENT APPLICATION

(11) **EP 3 797 763 A1**
(43) Date of publication of application: **31.03.2021**
(21) Application number: 19806677.1
(22) Date of filing: 22.05.2019
(51) Int. Cl.: A61K 31/047, A23L 33/125, A61P 1/00, A61P 1/14, A61P 3/02, A61P 3/04, A61P 5/00

(54) **GIP ELEVATION INHIBITOR**

(30) Priority: 23.05.2018 JP 2018098734
(71) Applicant: Kao Corporation, Chuo-ku, Tokyo 103-8210 (JP)
(72) Inventor: TAKAMURA, Akihiro, Haga-gun, Tochigi 321-3497 (JP); KOGA, Yoshitaka, Haga-gun, Tochigi 321-3497 (JP); HASHIZUME, Kohjiro, Haga-gun, Tochigi 321-3497 (JP); MORI, Takuya, Haga-gun, Tochigi 321-3497 (JP)
(74) Representative: Vossius & Partner Patentanwälte Rechtsanwälte mbB
(86) International application number: PCT/JP2019/020290
(87) International publication number: WO 2019/225653

(57) **Abstract**

Provided is a GIP elevation inhibitor useful as, e.g., a pharmaceutical product and food. The GIP elevation inhibitor comprises mannitol as an active ingredient.

## Description

### Field of the Invention

The present invention relates to a GIP elevation inhibitor.

### Background of the Invention

GIP (gastric inhibitory polypeptide or glucose-dependent insulinotropic polypeptide) is one of gastrointestinal hormones belonging to the glucagon/secretin family. GIP is secreted from K cells present in the small intestine when lipids and sugars are ingested; promotes secretion of insulin in pancreatic β cells; and enhances uptake of sugars and lipids in the adipose tissue. GIP is known to have a gastric-acid secretion inhibitory action and a gastric motility inhibitory action (Non Patent literatures 1 to 3). It is further shown that energy metabolism of a GIP-receptor deficient mouse is promoted in comparison with that of a wild-type mouse (Non Patent literature 4). It is reported that if energy metabolism is decreased, it becomes difficult to relive accumulated fatigue (Patent Literature 1).

Because of this, inhibition of GIP elevation is considered effective for, e.g., postprandial digestion promotion, stomach heaviness relief, energy metabolism enhancement, obesity prevention or improvement, and anti-fatigue.

According to researches so far conducted, 3-bromo-5-methyl-2-phenylpyrazolo[1,5-a]pyrimidin-7-ol (BMPP) is known as a GIP function inhibitory substance; guar gum and its derivatives are known as a substance that inhibits postprandial secretion of GIP (Patent Literature 2, Non Patent literatures 5 to 10); and recently, a GIP-receptor antagonist, (Pro3)-GIP has been also known. However, these substances are unsatisfactory in view of safety and effectiveness.

Meanwhile, mannitol has been reported for example, to inhibit coaggregation of oral bacteria (Patent Literature 3) and to promote vascular endothelial cell growth (Patent Literature 4).

However, no reports have been made on the relationship between mannitol and GIP secretion.

Patent Literature 1: JP-A-2007-308468
Patent Literature 2: WO 01/87341
Patent Literature 3: JP-A-2005-232057
Patent Literature 4: JP-A-H06-025001
Non Patent literature 1: Brown JC et al. Canadian J. Physiol. Pharmacol. 1969,47: 113-114
Non Patent literature 2: Falko JM et al., J. Clin. Endocrinol. Metab., 1975,41: 260-265
Non Patent literature 3: Toshitsugu Oda et al., Digestive tract function and pathology ("Shoukakan Kinou to Byoutai"), 1981, CHUGAI-IGAKUSHA, P 205-216
Non Patent literature 4: Miyawaki K et al., Nat. Med. 2002 Jun; 8 (7): 738-742
Non Patent literature 5: Gagenby S J et al., Diabet. Med. 1996 Apr; 13 (4): 358-64
Non Patent literature 6: Ellis PR et al., Br. J. Nutr. 1995 Oct; 74 (4): 539-56
Non Patent literature 7: Simoes NunesC et al., Reprod. Nutr. Dev. 1992; 32 (1): 11-20
Non Patent literature 8: Morgan LM et al., Br. J. Nutr. 1990 Jul; 64 (1): 103-10
Non Patent literature 9: Requejo F et al., Diabet. Med. 1990 Jul; 7 (6): 515-20
Non Patent literature 10: Morgan et al., Br. J. Nutr. 1985 May; 53 (3): 467-75

### Summary of the Invention

The present invention provides the following.
(1) A GIP elevation inhibitor comprising mannitol as an active ingredient.
(2) A food for GIP elevation inhibition, comprising mannitol as an active ingredient.
(3) A stomach heaviness reliever comprising mannitol as an active ingredient.
(4) A food for stomach heaviness reliever, comprising mannitol as an active ingredient.
(5) An energy metabolism enhancer comprising mannitol as an active ingredient.
(6) A food for energy metabolism enhancement, comprising mannitol as an active ingredient.
(7) An anti-fatigue agent comprising mannitol as an active ingredient.
(8) A food for anti-fatigue comprising mannitol as an active ingredient.
(9) Use of mannitol for producing a GIP elevation inhibitor.
(10) Use of mannitol for producing a food for GIP elevation inhibition.
(11) Use of mannitol for producing a stomach heaviness reliever.
(12) Use of mannitol for producing a food for relief of stomach heaviness.
(13) Use of mannitol for producing an energy metabolism enhancer.
(14) Use of mannitol for producing a food for energy metabolism enhancement.
(15) Use of mannitol for producing an anti-fatigue agent.
(16) Use of mannitol for producing a food for anti-fatigue.
(17) Mannitol for use in GIP elevation inhibition.
(18) Mannitol for use in stomach heaviness relief.
(19) Mannitol for use in energy metabolism enhancement.
(20) Mannitol for use in anti-fatigue.
(21) Non-therapeutic use of mannitol for inhibiting GIP elevation.
(22) Non-therapeutic use of mannitol for relieving stomach heaviness.
(23) Non-therapeutic use of mannitol for enhancing energy metabolism.
(24) Non-therapeutic use of mannitol for anti-fatigue.
(25) A method for inhibiting GIP elevation, comprising administration of mannitol.
(26) A method for relieving stomach heaviness, comprising administration or ingestion of mannitol.
(27) A method for energy metabolism enhancement, comprising administration or ingestion of mannitol.
(28) A method for anti-fatigue, comprising administration or ingestion of mannitol.

### Brief Description of the Drawings

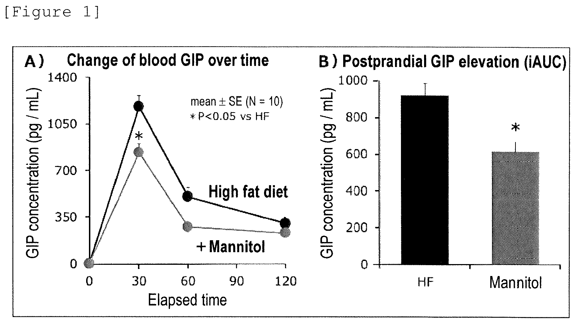

[Figure 1] Figure 1 shows inhibition of GIP elevation by D-mannitol in a single dietary test, where A) represents change in blood GIP concentration over time; and B) represents area under the curve of blood GIP concentration up to 120 minutes after administration.

### Detailed Description of the Invention

The present invention relates to a GIP elevation inhibitor that can be used in pharmaceutical products, foods and so on.

The present inventors conducted studies on materials capable of suppressing GIP elevation. As a result, they found that mannitol inhibits GIP elevation.

The GIP elevation inhibitor of the present invention has an excellent GIP elevation inhibitory action and high safety, and thus is useful as pharmaceutical products, foods and so on.

In the specification, "mannitol" is a sugar alcohol of mannose, and also called as mannite. As the mannitol to be used in the present invention, D-mannitol is preferable.

Mannitol may be a substance chemically synthesized or an extract from a natural product. D-mannitol is a substance widely distributed in the plant kingdom including brown alga, celery of vegetables, mushrooms and molds (*Aspergillus*), and can be easily extracted from plants, for example, manna and kelp. Industrially, D-mannitol is obtained by electrolytic reduction or catalytic reduction of D-glucose or an invert sugar under alkaline conditions, followed by epimerization, reduction, and removal of impurities.

In the present invention, a commercially available product (for example, "Mannitol" (Wako Pure Chemical Industries Ltd.)), can be also used.

As shown in Examples described later, mannitol has an activity to significantly inhibit GIP elevation. Accordingly, mannitol can serve as a GIP elevation inhibitor and be used for producing it. The inhibition of GIP elevation results in an effect to inhibit promotion of insulin secretion in pancreatic β cells, and enhancement of uptake of sugars and lipids in the adipose tissue, an effect to relieve inhibition of gastric acid secretion and inhibition of stomach motility (Non Patent literatures 1 to 3), an effect to enhance energy metabolism (Non Patent literature 4) and an effect to reduce fatigue (Patent Literature 1). Because of those, mannitol can be used as a postprandial digestion promoter, a stomach heaviness reliever, a promoter of gastric acid secretion, an energy metabolism enhancer and further, an anti-fatigue agent; and used for producing these.

Mannitol can be used for inhibiting GIP elevation, relieving stomach heaviness, enhancing energy metabolism and further for ant-fatigue. Such use may be use in humans or non-human animals or specimens derived from these and may be either therapeutic use or non-therapeutic use. Note that, the "non-therapeutic" does not conceptually include a medical practice, namely, does not conceptually include a method of surgically operating, treating or diagnosing humans, and more specifically, does not conceptually include a method for surgically operating, treating or diagnosing humans by a doctor or a person instructed by a doctor.

In the present invention, the "inhibition of GIP elevation" refers to inhibition of an elevation of GIP which is secreted from K cells present in the small intestine when a meal containing lipid and sugar, preferably a meal that is high in lipids is ingested. Namely, the "inhibition of GIP elevation" in the specification preferably refers to inhibition of postprandial GIP elevation. The "GIP elevation inhibitory action" in the specification conceptually includes both a GIP secretion inhibitory effect to inhibit GIP elevation by inhibiting GIP secretion from K cells, and a GIP lowering action to inhibit GIP elevation by reducing blood GIP concentration.

In the present invention, the "GIP elevation inhibitory action" can be determined based on the action to inhibit GIP elevation caused by intake of a meal containing lipid and sugar, preferably meal that is high in lipids. For example, the amount of blood GIP secretion among a test group ingesting an arbitrary meal containing lipid and sugar, preferably meal that is high in lipids, is compared to that of a control group ingesting the arbitrary meal containing lipid and sugar, preferably meal that is high in lipids. If the amount of blood GIP secretion among a test group is observed to be low compared to that of a control group, a test substance can be evaluated to have a GIP elevation inhibitory effect. In evaluation, it is not necessary to use a statistical technique; however, it is preferable to evaluate the efficacy by a statistical test to determine if there is significant difference.

The GIP elevation inhibitor, stomach heaviness reliever, energy metabolism enhancer and anti-fatigue agent of the present invention (hereinafter also referred to as "GIP elevation inhibitor and so on") can serve as a pharmaceutical product, quasi drug and food for humans or animals producing each effect such as a GIP elevation inhibitory effect, a postprandial digestion promotion effect, a stomach heaviness relieving effect, gastric acid secretion promoting effect, an energy metabolism enhancement effect, and further an anti-fatigue effect; and further serve as a material or a preparation to be blended and used in such pharmaceutical product, quasi drug and food.

Note that, examples of the food include foods, functional foods, specified health foods, foods for patients, and supplements, foods with functional claims which have a concept of promotion of postprandial digestion, relief of stomach heaviness, promotion of gastric acid secretion, enhancement of energy metabolism or anti-fatigue, with labels of the concepts as needed.

When the GIP elevation inhibitor and other related materials of the present invention are used as pharmaceutical products (including quasi drug), the pharmaceutical products may be administered in arbitrary dosage form. Examples of the dosage forms include oral administration using, for examples, a tablet, a capsule, a granule, a powder and a syrup, and parenteral administration using, for example, an injection, a suppository, an inhalant, a transdermal patch and a topical agent. Oral administration is preferable as a dosage form.

These pharmaceutical preparations having various dosage forms as mentioned above can be prepared by using mannitol of the present invention singly or appropriately in combination with other pharmaceutically acceptable additives such as an excipient, a binder, an extender, a disintegrant, a surfactant, a lubricant, a dispersant, a buffer, a preservative, a corrective agent, a flavor, a coating agent, a carrier and a diluent.

In case the GIP elevation inhibitor and other related materials of the present invention are used as food, examples of the food forms include various food compositions such as bread, cake, noodle, confectionery, jelly, a frozen food, ice cream, dairy product and beverage, as well as the same forms as the above-mentioned preparations for oral administration (e.g., a tablet, a capsule, a syrup).

The foods having various forms can be prepared by using mannitol singly or appropriately in combination with other food materials and additives such as a solvent, a softener, an oil, an emulsifier, an antiseptic, a flavor, a stabilizer, a colorant, an antioxidant, a moisturizer and a thickener.

When the GIP elevation inhibitor and the like of the present invention are used as a pharmaceutical product, quasi drug or food, or as a preparation to be blended and used in these, the content of mannitol is preferably 0.1 mass% or more and more preferably 1 mass% or more; and preferably 100 mass% or less and more preferably 20 mass% or less. Further, the content is preferably from 0.1 to 100 mass% and more preferably from 1 to 20 mass%.

The dose or intake of the GIP elevation inhibitor and other related materials of the present invention may vary depending on the condition, body weight, gender, age or other factors of the subject. In the case of oral administration or intake, the dose or intake of mannitol per adult/day is preferably 0.003 g or more and more preferably 0.03 g or more; and preferably 4 g or less and more preferably 3 g or less. The doses or intake is preferably from 0.003 to 4 g and more preferably from 0.3 to 3 g.

The GIP elevation inhibitor and other related materials of the present invention are preferably administered or ingested during or before eating/feeding and particularly preferably, within 5 to 30 minutes before eating/feeding. The subject for administration or intake is preferably a person having a fasting blood GIP value of 30 pg/mL or more, or a basal secretion volume of 30 mL/hour or less in a secretory function test of gastric juice. A person in need of promoting postprandial digestion, a person in need of relieving stomach heaviness, a person in need of promoting gastric acid secretion, a person with low energy metabolism, or a patient having or suspected to have a fatigue symptom (e.g., general malaise, slight fever, headache, lymphadenopathy, muscle pain, weakness, impairment involved in ability to think or concentrate, depression, sleep disturbance).

For the abovementioned embodiments, the following aspects of the present invention are disclosed.
<1> A GIP elevation inhibitor comprising mannitol as an active ingredient.
<2> A food for GIP elevation inhibition, comprising mannitol as an active ingredient.
<3> A stomach heaviness reliever comprising mannitol as an active ingredient.
<4> A food for relief of stomach heaviness, comprising mannitol as an active ingredient.
<5> An energy metabolism enhancer comprising mannitol as an active ingredient.
<6> A food for energy metabolism enhancement, comprising mannitol as an active ingredient.
<7> An anti-fatigue agent comprising mannitol as an active ingredient.
<8> A food for anti-fatigue comprising mannitol as an active ingredient.
<9> Use of mannitol for producing a GIP elevation inhibitor.
<10> Use of mannitol for producing a food for GIP elevation inhibition.
<11> Use of mannitol for producing a stomach heaviness reliever.
<12> Use of mannitol for producing a food for relief of stomach heaviness.
<13> Use of mannitol for producing an energy metabolism enhancer.
<14> Use of mannitol for producing a food for energy metabolism enhancement.
<15> Use of mannitol for producing an anti-fatigue agent.
<16> Use of mannitol for producing an anti-fatigue food.
<17> Mannitol for use in GIP elevation inhibition.
<18> Mannitol for use in relief of stomach heaviness.
<19> Mannitol for use in energy metabolism enhancement.
<20> Mannitol for use in anti-fatigue.
<21> (Non-therapeutic) use of mannitol for GIP elevation inhibition.
<22> (Non-therapeutic) use of mannitol for relieving stomach heaviness.
<23> (Non-therapeutic) use of mannitol for enhancing energy metabolism.
<24> (Non-therapeutic) use of mannitol for anti-fatigue.
<25> A method for inhibiting GIP elevation, comprising administration or ingestion of mannitol.
<26> A method for relieving stomach heaviness, comprising administration or ingestion of mannitol.
<27> A method for energy metabolism enhancement, comprising administration or ingestion of mannitol.
<28> A method for anti-fatigue, comprising administration or ingestion of mannitol.
<29> In <1>, <2>, <9>, <10>, <17>, <21> and <25>, the GIP elevation inhibition is inhibition of GIP elevation caused by intake of a meal comprising lipid and sugar, preferably a meal that is high in lipid.

### Examples

Now, the present invention will be more specifically described by way of Examples.

### Example 1

GIP elevation inhibitory activity of D-mannitol (single dietary test)

### (1) Method

Powdery feeds were prepared by using D-mannitol (manufactured by Kao Corp.) in accordance with the compositions listed in the following table and used as test samples.

**[Table 1]**

| Powdery feed composition (mass%) | | |
|---|---|---|
| | High fat diet group (HF) | D-mannitol group |
| Corn oil | 35.0 | 35.0 |
| Sucrose | 13.0 | 13.0 |
| Casein | 20.0 | 20.0 |
| Cellulose | 4.0 | 4.0 |
| Mineral | 3.5 | 3.5 |
| Vitamin | 1.0 | 1.0 |
| Potato starch | 23.5 | 23.5 |
| D-mannitol | - | 8.0 |
| Total | 100 | 108.0 |

Powdery feeds (Table 1) were weighed so as to have the same calories in each group and freely fed for 30 minutes to mice (C57BL/6J, CLEA Japan, Inc, male, 10-weeks old, n = 10) fasted for 18 hours or more. During this free feeding, 200 mg of the feed was given to the high fat diet (HF) group; whereas, 216 mg of the feed to the D-mannitol group. Note that the calorie of D-mannitol was calculated as 0. Before feeding, and 30, 60 and 120 minutes after feeding a powdery-feed test sample, the mice were anesthetized with isoflurane, blood was sampled from the orbit (heparinized hematocrit micro blood collection tube, manufactured by VITREX) and the blood GIP level was measured. The amount of GIP was measured in accordance with ELISA method (Rat/Mouse GIP (total) ELISA (Millipore)).

### (2) Results

A change in the blood GIP concentration over time is shown in Figure 1A, and relative area under the blood GIP concentration-time curve (AUC) is shown in Figure 1B.

Each value was shown by mean ± standard deviation. Statistically significant difference between the groups was obtained by applying Student's t-test to the HF group (*: p < 0.05).

In the D-mannitol group, the blood GIP concentration value at 30 minutes after intake of the sample was significantly low, compared to the HF group (Figure 1A). The area under the curve of GIP concentration up to 120 minutes after intake of the sample in the D-mannitol group was significantly low compared to the control group (Figure 1B).

From this, it is clear that D-mannitol has an inhibitory activity against GIP elevation.

## Claims

1. A GIP elevation inhibitor comprising mannitol as an active ingredient.

2. A food for GIP elevation inhibition, comprising mannitol as an active ingredient.

3. A stomach heaviness reliever comprising mannitol as an active ingredient.

4. A food for relief of stomach heaviness, comprising mannitol as an active ingredient.

5. An energy metabolism enhancer comprising mannitol as an active ingredient.

6. A food for energy metabolism enhancement, comprising mannitol as an active ingredient.

7. An anti-fatigue agent comprising mannitol as an active ingredient.

8. A food for anti-fatigue comprising mannitol as an active ingredient.

9. The inhibitor according to Claim 1 or the food according to Claim 2, wherein the GIP elevation inhibition is inhibition of GIP elevation caused by intake of a meal comprising lipid and sugar, preferably a meal that is high in lipids.

10. Use of mannitol for producing a GIP elevation inhibitor.

11. Use of mannitol for producing a food for GIP elevation inhibition.

12. Use of mannitol for producing a stomach heaviness reliever.

13. Use of mannitol for producing a food for relief of stomach heaviness.

14. Use of mannitol for producing an energy metabolism enhancer.

15. Use of mannitol for producing a food for energy metabolism enhancement.

16. Use of mannitol for producing an anti-fatigue agent.

17. Use of mannitol for producing a food for anti-fatigue.

18. Use according to Claim 10 or 11, wherein the GIP elevation inhibition is inhibition of GIP elevation caused by intake of a meal comprising lipid and sugar, preferably a meal that is high in lipids.

19. Mannitol for use in GIP elevation inhibition.

20. Mannitol for use in relief of stomach heaviness.

21. Mannitol for use in energy metabolism enhancement.

22. Mannitol for use in anti-fatigue.

23. Mannitol according to Claim 19, wherein the GIP elevation inhibition is inhibition of GIP elevation caused by intake of a meal comprising lipid and sugar, preferably a meal that is high in lipids.

24. Non-therapeutic use of mannitol for GIP elevation inhibition.

25. Non-therapeutic use of mannitol for relieving stomach heaviness.

26. Non-therapeutic use of mannitol for enhancing energy metabolism.

27. Non-therapeutic use of mannitol for anti-fatigue.

28. Use according to Claim 24, wherein the GIP elevation inhibition is inhibition of GIP elevation caused by intake of a meal comprising lipid and sugar, preferably a meal that is high in lipids

29. A method for inhibiting GIP elevation, comprising administration or ingestion of mannitol.

30. A method for relieving stomach heaviness, comprising administration or ingestion of mannitol.

31. A method for energy metabolism enhancement, comprising administration or ingestion of mannitol.

32. A method for anti-fatigue, comprising administration or ingestion of mannitol.

33. The method according to Claim 29, wherein the GIP elevation inhibition is inhibition of GIP elevation caused by intake of a meal comprising lipid and sugar, preferably a meal that is high in lipids.
